# EUROPÄISCHE PATENTSCHRIFT

(11) **EP 1 206 428 B1**
(45) Veröffentlichungstag und Bekanntmachung des Hinweises auf die Patenterteilung: **28.07.2004**
(21) Anmeldenummer: 00979636.8
(22) Anmeldetag: 29.11.2000
(51) Int. Cl.: C07C 43/13, A61K 7/06, A61K 7/48, A61K 31/045, A61K 31/075

(54) **VERWENDUNG VON HYDROXYETHERN IN KOSMETISCHEN UND PHARMAZEUTISCHEN ZUBEREITUNGEN**
USE OF HYDROXYETHERS IN COSMETIC AND PHARMACEUTICAL PREPARATIONS
UTILISATION D'HYDROXYETHERS DANS DES PREPARATIONS COSMETIQUES ET PHARMACEUTIQUES

(30) Priorität: 11.12.1999 DE 19959917
(43) Veröffentlichungstag der Anmeldung: 22.05.2002
(73) Patentinhaber: Cognis Deutschland GmbH & Co. KG, 40589 Düsseldorf (DE)
(72) Erfinder: ANSMANN, Achim, 40699 Erkrath (DE); KAWA, Rolf, 40789 Monheim (DE); NEUSS, Michael, 50997 Köln (DE)
(86) Internationale Anmeldenummer: PCT/EP2000/011938
(87) Internationale Veröffentlichungsnummer: WO 2001/042180

(56) Entgegenhaltungen:
- EP-A- 1 060 740
- DE-A- 2 535 802
- DE-A- 19 736 121
- US-A- 4 832 868

## Beschreibung

### Gebiet der Erfindung

Die Erfindung betrifft die Verwendung von speziellen Hydroxyethem, welche durch Ringöffnung von Olefinepoxiden mit Alkoholen und/oder Polyolen oder Wasser erhältlich sind, als Ölkörper in kosmetischen und/oder pharmazeutischen Zubereitungen zur Verbesserung des Hautglättegefühls.

### Stand der Technik

Zur Herstellung kosmetischer bzw. pharmazeutischer Mittel, beispielsweise Cremes, Lotionen oder Salben, werden öllösliche Grundlagen benötigt, in die in vielfach verschiedene lipidlösliche Feststoffe gelöst vorliegen. Für diesen Zweck eignen sich eine Vielzahl von natürlichen und synthetischen Ölen, beispielsweise Mandel- oder Avocadoöl oder Esteröle auf Basis von kurzkettigen Triglyceriden. Den Ölkomponenten in den angesprochenen Mitteln kommt gleichzeitig auch eine pflegende Wirkung zu, die in unmittelbarem Zusammenhang mit der Hautfettung steht. Vom Konsumenten gewünscht sind Produkte, die ein nichtklebriges, möglichst rasch eintretendes und länger anhaltendes Gefühl der Hautglätte- und -geschmeidigkeit vermitteln, darüber hinaus reichhaltig sind und gute Hautverträglichkeiten zeigen.

Das subjektive Empfinden auf der Haut kann mit dem physikochemischen Parameter der Spreitung der Ölkörper auf der Haut korreliert und objektiviert werden, wie dies von U. Zeidler in **Fette, Seifen, Anstrichmitt. 87, 403 (1985)** dargestellt worden ist. Demnach lassen sich kosmetische Ölkörper in niedrig-spreitende (unter 300 mm²/10min), mittelspreitende (ca. 300 bis 1000 mm²/10min) und hochspreitende Öle (oberhalb 1000 mm²/10min) einteilen. Setzt man in einer vorgegebenen Formulierung als Ölkörper ein hochspreitendes Öl ein, so wird zwar sehr rasch das gewünschte Gefühl der Hautglättung erzielt, das Erlebnis dauert jedoch nicht lange an. Umgekehrt wird bei Verwendung niedrig-spreitender Öle ein nur wenig ausgeprägtes Glättegefühl erzielt, welches über einen längeren Zeitraum praktisch unverändert erhalten bleibt und daher ebenfalls unbefriedigend ist. Die naheliegende Kombination eines niedrig- und eines hochspreitenden Öles führt erstaunlicherweise nicht zur angestrebten additiven
Wirkung, man nimmt vielmehr subjektiv zu Beginn die starke Glättewirkung der schnellspreitenden Ölkomponente und dann ganz unabhängig den Effekt des langsam spreitenden Öles wahr. In der Praxis müssen daher üblicherweise komplexe, genau aufeinander abgestimmte Mischungen von Ölkörpem eingesetzt werden, die im Sinne einer Kaskade ihre Wirkung nacheinander entfalten ohne sich dabei gegenseitig negativ zu beeinflussen. Es liegt auf der Hand, daß die Entwicklung derartiger Systeme mit hohem technischen Aufwand verbunden und damit auch aus ökonomischer Sicht verbesserungswürdig ist.

Aus der DE 25 35 802 sind mono-alkylsubstituierte Hydroxyether und deren Einsatz in kosmetischen Zusammensetzungen bekannt. Die DE 197 36 121 betrifft ein einstufiges Verfahren zur Herstellung von α-Hydroxyethern. Die EP 1060 740 offenbart. Polyolpartialether.

Demzufolge hat die Aufgabe der vorliegenden Erfindung darin bestanden, neue Mittel zur Verfügung zu stellen, die ein nichtklebriges, möglichst rasch eintretendes und längeranhaltendes Gefühl der Hautglätte- und -geschmeidigkeit vermitteln, reichhaltig sind und über gute Hautverträglichkeiten verfügen. Darüber hinaus sollen sie ein gutes Lösungsverhalten für lipophile Feststoffe zeigen.

### Beschreibung der Erfindung

Gegenstand der Erfindung ist die Verwendung von Hydroxyethem der Formel (I), 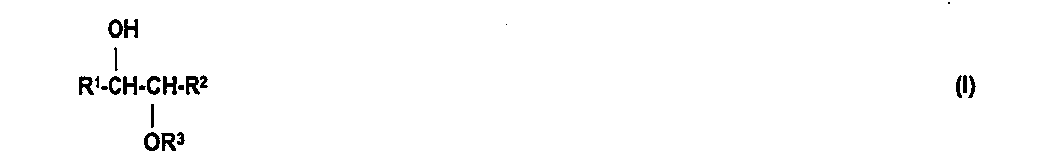 in der R¹ und R² für einen Alkylrest mit 4 bis 16 Kohlenstoffatomen steht, mit der Maßgabe, daß die Summe der Kohlenstoffatome von R¹ und R² im Bereich von 4 bis 16 liegt und R³ für einen Alkylund/oder Alkenylrest mit 1 bis 18 Kohlenstoffatomen und/oder den Rest eines Polyols mit 2 bis 18 Kohlenstoffatomen und 2 bis 10 Hydroxylgruppen stehen, als Ölkörper zur Herstellung von kosmetischen und/oder pharmazeutischen Zubereitungen zur Verbesserung des Hautglättegefühls.

Überraschenderweise wurde gefunden, daß diese Hydroxyether gemäß Formel I, welche durch Ringöffnung von Olefinepoxiden mit Alkoholen und/oder Polyolen oder Wasser erhältlich sind, in der Lage sind, verbesserte sensorischen Eigenschaften aufweisen und sich daher als Ölkörper in kosmetischen und/oder pharmazeutischen Zubereitungen eignen. Darüber hinaus zeigen sie ausgezeichnete Löseeigenschaften für lipophile Feststoffe, gute Hautverträglichkeiten und verbessern in erheblichem Maße die Photostabilität von UV-Lichtschutzfiltern.

### Hydroxyether (Epoxidringöffnungsprodukte)

Bei den Hydroxyethem der Formel I handelt es sich um bekannte Stoffe, die üblicherweise durch säurekatalysierte Umsetzung von endständigen oder innenständigen Olefinepoxiden mit aliphatischen Alkoholen hergestellt werden.

Typische Beispiele sind Ringöffnungsprodukte von α-Hexenepoxid, α-Octenepoxid, α-Decenepoxid, α-Dodecenepoxid, α-Hexadecenepoxid, α-Octadecenepoxid, i-Hexenepoxid, i-Octenepoxid, i-Decenepoxid, i-Dodecenepoxid, i-Hexadecenepoxid und/oder i-Octadecenepoxid mit Alkoholen mit 1 bis 18 Kohlenstoffatomen, vorzugsweise 1 bis 8 Kohlenstoffatomen, wie z.B. Laurylalkohol, Kokosfettalkohol, Myristylalkohol, Cetylalkohol, Cetearylalkohol, Stearylalkohol, Isostearylalkohol, Oleylalkohol, Elaidylalkohol, Petroselinylalkohol, Linolylalkohol und/oder Linolenylalkohol. Vorzugsweise werden Ringöffnungsprodukte von Octen-, Decen- und/oder Dodecenepoxiden eingesetzt. In einer besonders bevorzugten Ausführungsform der Erfindung können ebenfalls verzweigte und cyclische Alkohole mit 1 bis 18, vorzugsweise 1 bis 8 Kohlenstoffatomen eingesetzt werden.

Polyole, die im Sinne der Erfindung in Betracht kommen, besitzen vorzugsweise 4 bis 10 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Die Einsatzmenge der Hydroxyether kann 1 bis 60. vorzugsweise 2 bis 50 und insbesondere 3 bis 10 Gew.-% - bezogen auf die Endkonzentration - betragen.

### Lipophile Feststoffe

In den erfindungsgemäßen Ölkörpern können lipophile Feststoffe dispergiert vorliegen, die ausgewählt sind aus der Gruppe, die gebildet wird von UV-Lichtschutzfilter, Konsistenzgeber, Wachse, Fette, Stabilisatoren, Perlglanzwachse und/oder Partialglyceride.

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
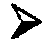 3-Benzylidencampher bzw. 3-Benzylidennorcampher und dessen Derivate, z.B. 3-(4-Methylbenzyliden)campher wie in der **EP 0693471 B1** beschrieben;
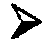 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
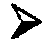 Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
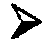 Ester der Salicylsäure, vorzugsweise Salicylsäure-2-ethylhexylester, Salicylsäure-4-isopropylbenzylester, Salicylsäurehomomenthylester;
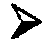 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
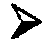 Ester der Benzalmalonsäure, vorzugsweise 4-Methoxybenzmalonsäuredi-2-ethylhexylester;
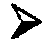 Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
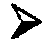 Propan-1,3-dione, wie z.B. 1-(4-tert.Butylphenyl)-3-(4'methoxyphenyl)propan-1,3-dion;
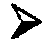 Ketotricyclo(5.2.1.0)decan-Derivate, wie in der **EP 0694521 B1** beschrieben.

Als wasserlösliche Substanzen kommen in Frage:
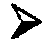 2-Phenylbenzimidazol-5-sulfonsäure und deren Alkali-, Erdalkali-, Ammonium-, Alkylammonium-, Alkanolammonium- und Glucammoniumsalze;
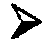 Sulfonsäurederivate von Benzophenonen, vorzugsweise 2-Hydroxy-4-methoxybenzophenon-5-sulfonsäure und ihre Salze;
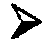 Sulfonsäurederivate des 3-Benzylidencamphers, wie z.B. 4-(2-Oxo-3-bomylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyf-4'-methoxydibenzoylmethan (Parsol 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Typische Beispiele für **Fette** sind Glyceride, d.h. feste oder flüssige (in diesem Fall oft auch als Öle bezeichnet) pflanzliche oder tierische Produkte, die im wesentlichen aus gemischten Glycerinestem höherer Fettsäuren bestehen, als Wachse kommen u.a. natürliche Wachse, wie z.B. Candelillawachs, Camaubawachs, Japanwachs, Espartograswachs, Korkwachs, Guarumawachs, Reis-keimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC) bezeichnet und folgen der allgemeinen Formel 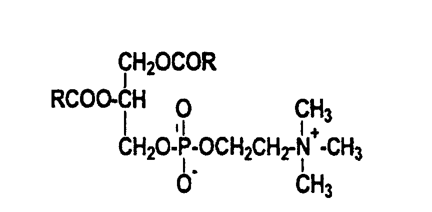 wobei R typischerweise für lineare aliphatische Kohlenwasserstoffreste mit 15 bis 17 Kohlenstoffatomen und bis zu 4 cis-Doppelbindungen steht. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als **Stabilisatoren** können Metallsalze von Fettsäuren, wie z.B. Magnesium-, Aluminium- und/oder Zinkstearat bzw. -ricinoleat eingesetzt werden.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether, Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Eine erfindungsgemäße Zubereitung aus Hydroxyether und lipophilen Fettstoffen weist die folgende Zusammensetzung auf - Mengenangaben bezogen auf die Endkonzentration - :
(a) 1 bis 60 Gew.-% Hydroxyether der Formel (I') 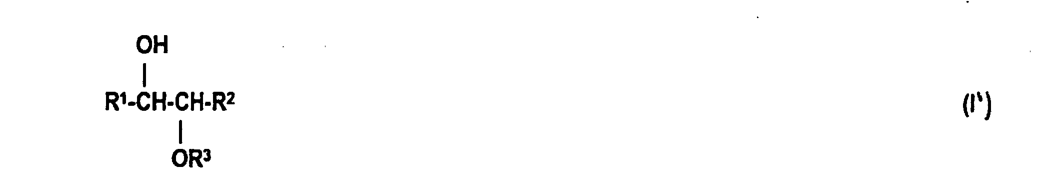 in der R¹ und R² für einen Alkylrest mit 4 bis 16 Kohlenstoffatomen steht, mit der Maßgabe, daß die Summe der Kohlenstoffatome von R¹ und R² im Bereich von 4 bis 16 liegt und R³ für einen Alkyl- und/oder Alkenylrest mit 1 bis 18 Kohlenstoffatomen steht und
(b) 0,1 bis 30 Gew.-% lipophile Feststoffe, mit der Maßgabe, daß sich die Mengenangaben mit Wasser und weiteren Hilfs- und Zusatzstoffen zu 100 Gew.-% ergänzen.

### Kosmetische und/oder pharmazeutische Zubereitungen

Die erfindungsgemäßen Mittel können zur Herstellung von kosmetischen und/oder pharmazeutischen Zubereitungen, wie beispielsweise Haarshampoos, Haarlotionen, Schaumbäder, Duschbäder, Cremes, Gele, Lotionen, alkoholische und wäßrig/alkoholische Lösungen, Emulsionen, Wachs/ Fett-Massen, Stiftpräparaten, Pudem oder Salben dienen. Diese Mittel können ferner als weitere Hilfs- und Zusatzstoffe milde Tenside, weitere Ölkörper, Emulgatoren, Überfettungsmittel, Verdickungsmittel (u.a. Polymere), Siliconverbindungen, Lecithine, Phospholipide, biogene Wirkstoffe, Deodorantien, Antitranspirantien, Antischuppenmittel, Filmbildner, Quellmittel, Antioxidantien, Hydrotrope, Konservierungsmittel, Insektenrepellentien, Selbstbräuner, Tyrosininhibitoren (Depigmentierungsmittel), Solubilisatoren, Parfümöle, Farbstoffe und dergleichen enthalten.

Typische Beispiele für geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als weitere **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettatkohoten, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, lsostearyloleat, Isostearylbehenat, lsostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. **DE 19756377 A1**); insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestem mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

Als **Emulgatoren** kommen beispielsweise nichtionogene Tenside aus mindestens einer der folgenden Gruppen in Frage:
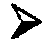 Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
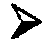 Block-Copolymere z.B. Polyethylenglycol-30 Dipolyhydroxystearate;
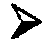 Alkyl- und/oder Alkenyloligoglykoside mit 8 bis 22 Kohlenstoffatomen im Alk(en)ylrest und deren ethoxylierte Analoga;
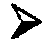 Anlagerungsprodukte von 1 bis 15 Mol Ethylenoxid an Ricinusöl und/oder gehärtetes Ricinusöl;
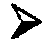 Anlagerungsprodukte von 15 bis 60 Mol Ethylenoxid an Ricinusöl und/oder gehärtetes Ricinusöl;
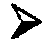 Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
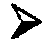 Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
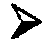 Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.
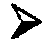 Mono-, Di- und Trialkylphosphate sowie Mono-, Di- und/oder Tri-PEG-alkylphosphate und deren Salze;
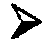 Wollwachsalkohole;
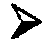 Polysiloxan-Polyalkyl-Polyether-Copolymere bzw. entsprechende Derivate;
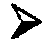 Polyalkylenglycole sowie
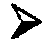 Pemulen-Typen, z.B. Pemulen TR-1, Pemulen TR-2 (Firma Goodrich).

Die **Anlagerungsprodukte von Ethylenoxid und/oder von Propylenoxid** an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

**Alkyl- und/oder Alkenyloligoglycoside,** ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete **Partialglyceride** sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als **Sorbitanester** kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete **Polyglycerinester** sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische.

Beispiele für weitere geeignete **Polyolester** sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Weiterhin können als Emulgatoren **zwitterionische Tenside** verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Schließlich kommen auch **Kationtenside** als Emulgatoren in Betracht, wobei solche vom Typ der Esterquats, vorzugsweise methylquatemierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Geeignete **Verdickungsmittel** sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, femer höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quatemierte Hydroxyethylcellulose, die unter der Bezeichnung Polymer JR 400® von Amerchol erhältlich ist, kationische Stärke, Copolymere von Diallylammoniumsalzen und Acrylamiden, quatemierte Vinylpyrrolidon/Vinylimidazol-Polymere, wie z.B. Luviquat® (BASF), Kondensationsprodukte von Polyglycolen und Aminen, quatemierte Kollagenpolypeptide, wie beispielsweise Lauryldimonium Hydroxypropyl Hydrolyzed Collagen (Lamequat®L/Grünau), quatemierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840** A sowie deren vemetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quatemierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und deren Ester, unvemetzte und mit Polyolen vemetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxyproylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in **Cosmetics & Toiletries Vol. 108, Mai 1993, Seite 95ff** aufgeführt.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil.** 91, 27 (1976).

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Kosmetische **Deodorantien** (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

Als **keimhemmende Mittel** sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)hamstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Famesol, Phenoxyethanol, Glycerinmonolaurat (GML), Diglycerinmonocaprinat (DMC), Salicylsäure-N-alkylamide wie z. B. Salicylsäure-n-octylamid oder Salicylsäure-n-decylamid.

Als **Enzyminhibitoren** sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als **Geruchsabsorber** eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern. Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilat, Irotyl und Floramat allein oder in Mischungen, eingesetzt.

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
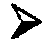 adstringierende Wirkstoffe,
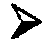 Ölkomponenten,
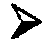 nichtionische Emulgatoren,
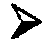 Coemulgatoren,
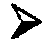 Konsistenzgeber,
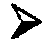 Hilfsstoffe wie z. B. Verdicker oder Komplexierungsmittel und/oder
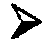 nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin.

Daneben können in Antitranspirantien übliche öllösliche und wasserlösliche Hilfsmittel in geringeren Mengen enthalten sein. Solche öllöslichen Hilfsmittel können z.B. sein:
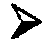 entzündungshemmende, hautschützende oder wohlriechende ätherische Öle,
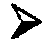 synthetische hautschützende Wirkstoffe und/oder
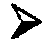 öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

Als **Antischuppenmittel** können Octopirox® (1-Hydroxy-4-methyl-6-(2,4,4-trimythylpentyl)-2-(1H)-pyridon-monoethanolaminsalz), Baypival, Pirocton Olamin, Ketoconazol®, (4-Acetyl-1-{-4-[2-(2.4-dichlorphenyl)r-2-(1H-imidazol-1-ylmethyl)-1,3-dioxylan-c-4-ylmethoxyphenyl}piperazin, Selendisulfid, Schwefel kolloidal, Schwefelpolyehtylenglykolsorbitanmonooleat, Schwefelrizinolpolyehtoxylat, Schwefelteer Destillate, Salicylsäure (bzw. in Kombination mit Hexachlorophen), Undexylensäure Monoethanolamid Sulfosuccinat Na-Salz, Lamepon® UD (Protein-Undecylensäurekondensat, Zinkpyrethion, Aluminiumpyrition und Magnesiumpyrithion / Dipyrithion-Magnesiomsulfat eingesetzt werden.

Gebräuchliche **Filmbildner** sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quatemäre Cellulose-Derivate, Kollagen, Hyaluronsäure bzw. deren Salze und ähnliche Verbindungen.

Als **Quellmittel** für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil.108, 95 (1993)** entnommen werden.

Neben den genannten löslichen Stoffen kommen für diesen Zweck auch unlösliche **Lichtschutzpigmente,** nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** sowie **Parfümerie und Kosmetik** 3 **(1999), Seite 11ff** zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), femer (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-Apalmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
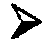 Glycerin;
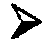 Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
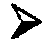 technische Oligoglyceringemische mit einem Eigenkondensationsgrad von 1,5 bis 10 wie etwa technische Diglyceringemische mit einem Diglyceringehalt von 40 bis 50 Gew.-%;
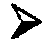 Methyolverbindungen, wie insbesondere Trimethylolethan, Trimethylolpropan, Trimethylolbutan, Pentaerythrit und Dipentaerythrit;
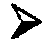 Niedrigalkylglucoside, insbesondere solche mit 1 bis 8 Kohlenstoffen im Alkylrest, wie beispielsweise Methyl- und Butylglucosid;
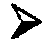 Zuckeralkohole mit 5 bis 12 Kohlenstoffatomen, wie beispielsweise Sorbit oder Mannit,
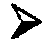 Zucker mit 5 bis 12 Kohlenstoffatomen, wie beispielsweise Glucose oder Saccharose;
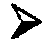 Aminozucker, wie beispielsweise Glucamin;
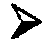 Dialkoholamine, wie Diethanolamin oder 2-Amino-1,3-propandiol.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-toluamid, 1,2-Pentandiol oder Ethyl Butylacetylaminopropionate in Frage, als **Selbstbräuner** eignet sich Dihydroxyaceton. Als **Tyrosinhinbitoren,** die die Bildung von Melanin verhindern und Anwendung in Depigmentierungsmitteln finden, kommen beispielsweise Arbutin, Kojisäure, Cumarinsäure und Ascorbinsäure (Vitamin C) in Frage.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Yiang-Yiang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedem-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel"** der Farbstoff**kommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

**Tabelle 1:**

| **Standardemulsionen - Mengenangaben in Gew.-% bezogen auf die Endkonzentration -** | | |
|---|---|---|
| **Standardemulsion** | **(I)** | **(II)** |
| Ceteareth-20 | 2 | 2 |
| Cetearyl Alcohol | 2 | 2 |
| Glyceryl Stearate | 4 | 4 |
| Ölkörper | 20 | 20 |
| Octyl Methoxycinamate | - | 4 |
| Butyl Methoxydibenzoylmethane | - | 4 |
| Glycerin | 5 | 5 |
| Carbomer | 0,2 | 0,2 |
| Wasser, Konservierung | ad 100 | |

### A) Subjektive Beurteilung der Hautglätte

Ein Panel bestehend aus 5 erfahrenen Personen testete Formulierungen auf Basis einer Standardmulsion (I), die verschiedene Ölkörpermischungen enthielten auf ihr subjektives Hautglättegefühl. Zugrunde gelegt wurde eine Skala zwischen 1 (kaum Glättung bzw. rasche Abnahme des Glättegefühls) und 6 (schnelles, gleichmäßiges Glättegefühl). Die Angaben in der Tabelle 2 stellen Mittelwerte dar. Die Beispiele 1 bis 6 sind erfindungsgemäß, die Beispiele V1 bis V9 dienen dem Vergleich.
A1: C8 Epo/n-Octanol (erfindungsgemäße Ölkomponente)
B1: Dicaprylyl Ether
B2. Cetearyl Isononanoate
B3: Isopropyl Myristate
B4: Dioctyl Cyclohexane
B5: Mandelöl
B6: Oleyl Erucate

**Tabelle 2:**

| **Kosmetische Formulierungen** | | | | |
|---|---|---|---|---|
| **Beispiel** | **Komponente 1** | **Komponente 2** | **Verhältnis** | **Hautglätte** |
| 1 | A1 | - | 100 : 0 | 5,7 |
| 2 | A1 | B1 | 50 : 50 | 5,4 |
| 3 | A1 | B2 | 60 : 40 | 5,5 |
| 4 | A1 | B3 | 40 : 60 | 5,8 |
| 5 | A1 | B4 | 50 : 50 | 5,4 |
| 6 | A1 | B5 | 80 : 20 | 5,2 |
| 7 | A1 | B6 | 80 : 20 | 5,3 |
| V1 | - | B1 | 0 : 100 | 3,0 |
| V2 | - | B2 | 0 : 100 | 3,2 |
| V3 | - | B3 | 0 : 100 | 2,0 |
| V4 | - | B4 | 0 : 100 | 2,4 |
| V5 | - | B5 | 0 : 100 | 1,3 |
| V6 | - | B6 | 0 : 100 | 1,5 |
| V7 | B1 | B2 | 50 : 50 | 3,2 |
| V8 | B3 | B6 | 80 : 20 | 2,9 |
| V9 | B4 | B5 | 80 : 20 | 2,2 |

Oberhalb eines Mittelwertes von 5,0 liegt ein ausgezeichnetes Hautglättegefühl vor, unterhalb von 5,0 ist das Ergebnis unbefriedigend.

### B) Lösevermögen von lipophilen Feststoffen

Löslichkeit von Octyl Triazone in:

| | |
|---|---|
| Dibutyl Adipate | 10 % |
| Dicaprylyl Ether | 0 % |
| Hexyl Laurate | 0 % |
| Cocoglycerides | 5 % |
| Dioctyl Malate | 10 % |
| C12-15 Alkyl Benzoate | 10 % |
| C12 Epo/Methanol | 40 % |
| C8 Epo/n-Octanol | 30 % |

### C) Verbesserung der Photostabilität

Die Photostabilität der UV-Filterkombination Octyl Methoxycinamate / Butyl Methoxydibenzoylmethane wurde in einer Standardemulsion (II) geprüft. Hierbei wurde der Einfluß auf die Photostabilität der erfindungsgemäßen Ölkomponente A1 im Vergleich zu den Ölen B1 - B6 nach der von der Firma Merck anläßlich des APV - Seminars vom 17. - 18. 9. 1997 in Fulda veröffentlichten Methode bestimmt.
Die Ergebnisse sind in der nachfolgenden Tabelle 3 zusammengefaßt. Hierbei wurde die Photostabilität der Emulsion auf Basis der erfindungsgemäßen Ölkomponente A1 auf 100% gesetzt und verglichen.

**Tabelle 3:**

| Kosmetische Formulierungen | | | | |
|---|---|---|---|---|
| Beispiel | Komponente 1 | Komponente 2 | Verhältnis | Photostabilität |
| 1 | A1 | - | 100 : 0 | 100% |
| 2 | A1 | B1 | 50 : 50 | 89% |
| 3 | A1 | B2 | 60 : 40 | 92% |
| 4 | A1 | B3 | 40 : 60 | 82% |
| 5 | A1 | B4 | 50 : 50 | 89% |
| 6 | A1 | B5 | 80 : 20 | 94% |
| 7 | A1 | B6 | 80 : 20 | 96% |
| V1 | - | B1 | 0 : 100 | 55% |
| V2 | - | B2 | 0 : 100 | 63% |
| V3 | - | B3 | 0 : 100 | 42% |
| V4 | - | B4 | 0 : 100 | 39% |
| V5 | - | B5 | 0 : 100 | 45% |
| V6 | - | B6 | 0 : 100 | 66% |
| V7 | B1 | B2 | 50 : 50 | 60% |
| V8 | B3 | B6 | 80 : 20 | 54% |
| V9 | B4 | B5 | 80 : 20 | 40% |

**Tabelle 4:**

| **Sonnenschutz-/ Pflegeemulsionen vom Typ O/W - Mengenangaben in Gew.-% bezogen auf die Endkonzentration-** | | | | | | | | | | | | | | | | | | | | | | |
|---|---|---|---|---|---|---|---|---|---|---|---|---|---|---|---|---|---|---|---|---|---|---|
| **Komponente (INCI)** | **1** | **2** | **3** | **4** | **5** | **6** | **7** | **8** | **9** | **10** | **11** | **12** | **13** | **14** | **15** | **16** | **17** | **18** | **19** | **20** | **21** | **22** |
| L = Lotion, C = Creme | L | C | C | L | C | L | L | C | L | C | L | C | L | L | C | L | C | L | L | L | L | C |
| Polyglyceryl-2 Dipolyhydroxystearate (and) Lauryl Glucoside (and) Glycerin | | | | 3.5 | | 4 | 4 | | | | | | 4 | 3 | 4 | 3 | 4 | | | | | 2 |
| Ceteareth-20 | 2 | | | | | | | | | | 2 | | | | | | | | | | 1 | |
| Polysorbate 60 | | 1 | | | | | | | | | 1 | | | | | | | | | | 1 | |
| PEG-30 Stearate | | 3 | | | | | | | | | | | | | | | 3 | | | | | |
| PEG-20 Glyceryl Stearate | 1 | | | | | | | | | | | | | | 2 | | | | | | | |
| Trilaureth-4 Phosphate | | | | | | | | | 2 | | | | | | | | | | | | 1 | |
| Sodium Cetearyl Sulfate | | | | 1 | | | | 1 | | | | | | 1 | .5 | | | | | 1 | | |
| Potassium Cetyl Phosphate | | | 1 | | | 1 | | | | 1 | | | 1 | | | | | | 1 | | | |
| Sodium Stearate | | | | | 1 | | 1 | | | | | | | | | 1 | | | | | | 1 |
| Cetearyl Glucoside + Ceteayl Alcohol | | | 5 | | 5 | | | | | 4 | | | 6 | | | | | | 5 | | | 4 |
| Polyglyceryl-3 Methylglucose Distearate | | | | | | | | 5 | | 3 | | 5 | | | | | | | | 4 | | |
| Glyceryl Stearate | 4 | | 4 | 6 | | | 4 | | | 6 | | 3 | | 6 | 8 | 6 | 8 | | | | 4 | |
| Myristyl Alcohol | 1 | | | 1 | | | | 2 | | | 4 | | 2 | | | | | | 2 | | 1 | |
| Cetearyl Alcohol | 1 | 6 | | | 5 | 2 | | | | | | | | | 2 | | 3 | | | 1 | 1 | 6 |
| PVP / Hexadecene Copolymer | 1 | 1 | | | | | | | | | | | | | | | 2 | | | | | |
| Lanolin Alcohol | | | | | .5 | .5 | | | | | | | | | | | | | | | | |
| Lanolin | | | | | | | 5 | | | | | | | | | | 4 | | | | | |
| **C12 Epo/Methanol** | | 4 | 4 | | 8 | 4 | | 5 | 10 | | | 4 | | 4 | 8 | 6 | 6 | | | 4 | 4 | |
| **C8 Epo/Octanol** | 6 | | | 6 | | 2 | 5 | 5 | | 5 | 8 | | 10 | 4 | 2 | | | 10 | 8 | | | 5 |
| Myristyl Lactate | | | | | | | | | | | | 4 | | | | | | | | | | |
| Propylene Glycol Dicaprylate/Dicaprate | | | | | 5 | | | | | | | 6 | | | | | 5 | | | 5 | | |
| Cocoglycerides | 5 | | | | | 6 | | 6 | | | | 5 | | 5 | | | | 7 | | | 10 | 8 |
| C12/15 Alkyl Benzoate | | | 3 | | | | | | 8 | | | | | | | 5 | | | 3 | 3 | | |
| Dicaprylyl Carbonate | | 2 | | | | 4 | | | | | | | | | | | | | | | | |
| Dicaprylyl Ether | | | 3 | | | | | | 2 | 3 | | | | | | 2 | | 2 | | | | |
| Cyclomethicone | 4 | | 1 | | 5 | | | | | | 2 | | 2 | | | 1 | | | | | | |
| Cetyl Dimethicone | | 1 | | | 2 | | | | | | | | | 1 | | 1 | | | | | | 3 |
| Dimethicone | | 2 | | | | | 4 | | | | | | | | | 1 | | | | | | |
| Octyl Stearate | | | 2 | | 2 | | 4 | | | | 7 | | 2 | | | | | | | | | |
| Oleyl Erucate | | | | | 3 | 2 | | | | 5 | | | 2 | | | | | | | | 1 | |
| Mineral Oil | | | | 9 | | | | | | | | | | | 10 | | | | | | | |
| Butyl Adipate | | | 1 | | | | | | | 2 | | | | | | | 5 | | 4 | | | 3 |
| Octyldodecanol | | | | | | | | | | | | | 3 | 5 | | | | | | | | |
| Hexyldecanol + Hexyldecyllaurate | | 5 | | | | | | | | 5 | | | | | | | | | | 2 | | |
| Almond Oil | | | 2 | | | | 1 | | | | | | | | | | 2 | | 3 | | | 2 |
| Panthenol | 1 | | | | | | | | | | | | | | | | | | | | | |
| Bisabolol | 0,2 | | | | | | | | | | | | | | | | | | | | | |
| Tocopherol / Tocopheryl Acetate | 1 | | | | | | | | | | | | | | | | | | | | | |
| Chitosan | | | | | | | | | 0.2 | | | | | 0.2 | | | | | | 0.2 | | |
| Phenylbenzimidazole Sulfonic Acid (Sodium Salt) | 2 | | 2.2 | | 3 | 3 | | | | | | | | | | | | | | | | |
| Octocrylene | 3 | 5 | | | | | | | | 4 | | | | | | | | | | | | |
| Benzophenone-3 | | | | 4 | 3 | | | | | | | | | | | | | | | | | |
| 4-Methylbenzylidene Camphor | 2 | | 3 | 3 | | 2 | 2 | | | | | | | | | | | | | | | |
| Octyl Salicylate | | | | | | | | | 10 | 7 | | | | | | | | | | | | |
| Isoamyl p-Methoxycinnamate | | 7.5 | 6 | 6 | | | | | | | | | | | | | | | | | | |
| Octyl Methoxycinnamate | | | | | | 7.5 | 4 | 5 | | | | | | | | | | | | | | |
| Octyl Triazone | 2 | | | | 2.5 | | | 3 | | | | | | | | | | | | | | |
| Butyl Methoxydibenzoytmethane | | 1 | 1 | | | | 2 | 2 | 2 | | | | | | | | | | | | | |
| Zinc Oxide (micronisiert) | 10 | | | | | 10 | | | | 6 | | | | | | | | | | | | |
| Titanium Dioxide (oberflächenbehandelt) | | | 10 | 6 | 5 | | 3 | | | | | | | | | | | | | | | |
| Magnesium Aluminium Silicates | | | 1 | | | | | | 1 | | | | | | | | | | | 1 | | |
| Xanthan Gum | | | .5 | | | | | | .5 | | | | | .2 | | | | | | .5 | | |

| **Sonnenschutz-/ Pflegeemulsionen vom Typ O/W** | | | | | | | | | | | | | | | | | | | | | | |
|---|---|---|---|---|---|---|---|---|---|---|---|---|---|---|---|---|---|---|---|---|---|---|
| **Komponente (INCI)** | **1** | **2** | **3** | **4** | **5** | **6** | **7** | **8** | **9** | **10** | **11** | **12** | **13** | **14** | **15** | **16** | **17** | **18** | **19** | **20** | **21** | **22** |
| Carbomer | | .5 | | | .2 | .2 | | .5 | | .3 | .2 | | .3 | .2 | .3 | | .5 | .2 | .1 | | .3 | |
| Acryylates/C10-30 Alkyl Acrylate Crosspolymer | | | | .2 | | | | | | | | | | | | | | | .2 | | | |
| Ethanol | | | | | | | | | | 10 | | | 5 | | 8 | | | | | | | 10 |
| Butylenc Glycol | | 2 | | 4 | 3 | | 2 | 5 | 2 | | | 5 | | 2 | 3 | 3 | | | | | 8 | |
| Glycerin | 5 | 5 | 5 | | 3 | 3 | 2 | | 4 | 5 | 3 | 2 | 4 | 3 | 3 | | 7 | 5 | 3 | 5 | | 5 |
| Wasser, Konservierungsmittel, NaOH (pH 5-6) | ad 100 | | | | | | | | | | | | | | | | | | | | | |
| Sonnenschutzemulsionen (1-10); Pflegeemulsionen (11-22) | | | | | | | | | | | | | | | | | | | | | | |

**Tabelle 5:**

| **Sonnenschutz-/ Pflegeemulsionen vom Typ W/O - Mengenangaben in Gew.-% bezogen auf die Endkonzentration -** | | | | | | | | | | | | | | | | | | | | | | |
|---|---|---|---|---|---|---|---|---|---|---|---|---|---|---|---|---|---|---|---|---|---|---|
| **Komponente (INCI)** | **23** | **24** | **25** | **26** | **27** | **28** | **29** | **30** | **31** | **32** | **33** | **34** | **35** | **36** | **37** | **38** | **39** | **40** | **41** | **42** | **43** | **44** |
| L = Lotion. C = Creme | C | L | C | L | C | L | L | L | C | C | C | L | C | L | L | C | L | L | L | L | C | C |
| Polyglyceryl-2 Dipolyhydroxystearate | 4 | 2 | | 3 | 3 | | | 2 | 1 | | | 3 | | 5 | | | 3 | | 4 | | | 1 |
| Glyceryl Oleate | | | 2 | | | | | | | | | | 1 | | | | | | | | | 3 |
| Polyglyccryl-3 Düsostearate | | | 4 | | 3 | | | | 4 | 1 | 3 | | | | | 4 | | | 1 | | 3 | |
| Cetyl Dimethicone Copolyol | | 1 | | | | | 4 | | | | | | | | 1 | | | | | | 2 | |
| Methyl Glucose Dioleate | 2 | | | | | | | | | | 3 | | | | 3 | | | | | 2 | | |
| PEG-30 Dipolyhydroxystearate | | | | | | | | 1 | | | | 2 | | | | | | | | | | 1 |
| Diisostearoyl Polyglyceryl-3 Diisostearate | | | | | | 4 | | 2 | | | | | 3 | | | | | 4 | | | | |
| Sorbitan Sesquioleate | | | | 2 | | | | | | | | | | | | | 3 | | | | | |
| Dicocoyl Pentaerythrityl Distearyl Citrate | | | | 2 | | | | | | | | | | | | 4 | | | | | | |
| PEG-7 Hydrogenated Castor Oil | | | | | | | | | | 4 | | | | | | | | | | 4 | | |
| Zinc Stearate | 2 | 1 | | 1 | 1 | | | 1 | 1 | 1 | | | 2 | 2 | 1 | 1 | 1 | | 1 | 1 | | 1 |
| Microcrystalline Wax | | | 5 | | | 2 | | | | | 5 | | | | | 4 | | 1 | | | 4 | |
| Beeswax | 4 | 1 | | 1 | | | | 5 | 4 | 7 | | | 4 | | 2 | | 2 | 1 | 1 | 2 | | 5 |
| Cetearyl Glucoside | | | | | 1 | | | | | | | | | .5 | | | | | | | | |
| Isostearic Acid | | | 1 | | 1 | 1 | 1 | | | | | | | | | | | | 1 | | | |
| PVP / Hexadecene Copolymer | | | | | 1 | | | | | | | | | | | | | 1 | | | | |
| Lanolin Alcohol | 1 | | | | | | | | | | 1 | | | | | | | | | | | |
| Lanolin | | | 5 | | | | | | | 4 | | | 7 | 3 | | | | | | | | |
| C12 Epo/Methanol (erfindungsgernäße Ölkomponente) | 5 | 8 | 3 | 4 | | | | 8 | 5 | | 8 | | 10 | | | 10 | | 7 | 2 | 4 | 10 | 2 |
| C 8 Epo/Octanol (erfindungsgemäße Ölkomponente) | | | 4 | | 6 | 8 | 5 | | 5 | 4 | | 12 | | 8 | 8 | | 8 | | 7 | | | 5 |
| Myristyl Lactate | | | 3 | | | | | | | 1 | | | 2 | | | | | | | | | |
| Propylene Glycol Dicaprylate/Dicaprate | | | | | 3 | | | 4 | | | | | | | | | | | | | | |
| Cocoglycerides | 6 | | | | 3 | 6 | | | | | 8 | 4 | | 3 | | 5 | | | | 5 | 4 | |
| C12/15 Alkyl Benzoate | | | | 5 | | | 5 | | | | | | | 5 | | | 7 | | | | | |
| Dicaprylyl Carbonate | | 4 | | | | 2 | | | | | 5 | 3 | | 2 | | | | 3 | | | | |
| Dicaprylyl Ether | 3 | | | | 4 | | 5 | | 4 | 2 | | | 3 | | 2 | | | 3 | | | | |
| Cyclomethicone | | 3 | | | | | 2 | | | | | | 4 | | 2 | | | | | | | |
| Cetyl Dimethicone | | | 1 | | 2 | | | | | | | | | | 1 | | | | | | | |
| Dimethicone | | | | 4 | | | | 3 | | | | | | | 1 | | | | 4 | | | |
| Octyl Stearate | | | | | | | | | | 2 | | | | | | | | | | | | 10 |
| Oleyl Erucate | | | 4 | | | 2 | | | | | | | | | 3 | | | | | | | |
| Dioctyl Malate | | | | 2 | | | | 2 | | 6 | | | | | 1 | | | | | 5 | 4 | |
| Mineral Oil | | | | | 4 | | | | | | | | | | | | | 9 | | | | |
| Butyl Adipate | | | 2 | 4 | | | | | | 3 | | | | | | 3 | 3 | | 2 | 2 | | |

| **Sonnenschutz-/ Pflegeemulsionen vom Typ W/O** | | | | | | | | | | | | | | | | | | | | | | |
|---|---|---|---|---|---|---|---|---|---|---|---|---|---|---|---|---|---|---|---|---|---|---|
| **Komponente (INCI)** | **23** | **24** | **25** | **26** | **27** | **28** | **29** | **30** | **31** | **32** | **33** | **34** | **35** | **36** | **37** | **38** | **39** | **40** | **41** | **42** | **43** | **44** |
| Octyldodecanol | | | 3 | | | | | 8 | | | | | | | 2 | | | | | 5 | | |
| Hexyldecanol (and) Hexyldecyllaurate | | 11 | | | | 4 | | | 9 | | | | | | | | | | 6 | | | 3 |
| Almond Oil | | | | | 1 | | 5 | | | | | | | 2 | | | | | | | | |
| Panthenol | 1.0 | | | | | | | | | | | | | | | | | | | | | |
| Bisabolol | 0.2 | | | | | | | | | | | | | | | | | | | | | |
| Tocopherol / Tocopheryl Acetate | 1.0 | | | | | | | | | | | | | | | | | | | | | |
| Chitosan | 0.2 | | | | | | | | | | | | | | | | | 0.3 | | | | |
| Phenylbenzimidazole Sulfonic Acid (Sodium Salt) | | 1 | | 3 | | | | | | | | | | | | | | | | | | |
| Ostocrylene | 5 | | | | 4 | | | | | 6 | | | | | | | | | | | | |
| Benzophenone-3 | | | 2 | 4 | | | 2 | | | | | | | | | | | | | | | |
| 4-Methylbenzylidene Camphor | 3 | 3 | | | | | | 4 | | 3 | | | | | | | | | | | | |
| Octyl Salicylate | | | | | | | | | | | | | | | | | | | | | | |
| Isoamyl p-Methoxycinnamate | | 7.5 | | | | | | | 5 | | | | | | | | | | | | | |
| Octyl Methoxycinnamate | | | 6 | 6 | | 7.5 | 7.5 | | | | | | | | | | | | | | | |
| Octyl Triazone | 2 | | | | 2.5 | | | 1 | 3 | | | | | | | | | | | | | |
| Butyl Methoxydibenzoytmethane | 1 | 2 | | | | | | 1 | 2 | 2 | | | | | | | | | | | | |
| Zinc Oxide (micronisiert) | | | | | 5 | 6 | | | | | | | | | | | | | | | | |
| Titanium Dioxide (oberflächenbehandelt) | | | 10 | | | | 4 | | | | | | | | | | | | | | | |
| Ethanol | | | | | | | | | | 8 | | | | | 8 | | 10 | | | | | |
| Butylene Glycol | | | 6 | 3 | | 2 | 5 | | | | | 5 | | | 3 | 3 | | | | 8 | 2 | 1 |
| Glycerin | 5 | 3 | 3 | | 5 | 3 | | 4 | 10 | 4 | 3 | | 4 | 6 | 3 | | 4 | 5 | 5 | | 3 | 5 |
| Wasser, Konservierungsmittel | ad 100 | | | | | | | | | | | | | | | | | | | | | |
| Sonnenschutzemulsionen (23-32); Pflegeemulsionen (33-44) | | | | | | | | | | | | | | | | | | | | | | |

## Patentansprüche

1. Verwendung von Hydroxyether der Formel **(I)**, 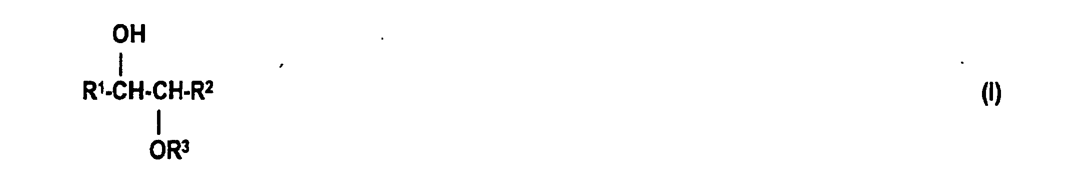 in der R¹ und R² für einen Alkylrest mit 4 bis 16 Kohlenstoffatomen steht, mit der Maßgabe, daß die Summe der Kohlenstoffatome von R¹ und R² im Bereich von 4 bis 16 liegt und R³ für einen Alkylund/oder Alkenylrest mit 1 bis 18 Kohlenstoffatomen und/oder den Rest eines Polyols mit 2 bis 18 Kohlenstoffatomen und 2 bis 10 Hydroxylgruppen stehen, als Ölkörper zur Herstellung von kosmetischen und/oder pharmazeutischen Zubereitungen zur Verbesserung des Hautglättegefühls.

2. Verwendung nach den Anspruch 1, **dadurch gekennzeichnet, daß** in den Ölkörpem lipophile Feststoffe dispergiert sind, die ausgewählt sind aus der Gruppe, die gebildet wird von UV-Lichtschutzfilter, Wachse, Fette, Stabilisatoren, Perlglanzwachse, Polymere und/oder Partialglyceride.

3. Mittel nach mindestens einem der Ansprüche 1 bis 2, **dadurch gekennzeichnet, daß** sie - bezogen auf die Endkonzentration -
(a) 1 bis 60 Gew.-% Hydroxyether der Formel (I') 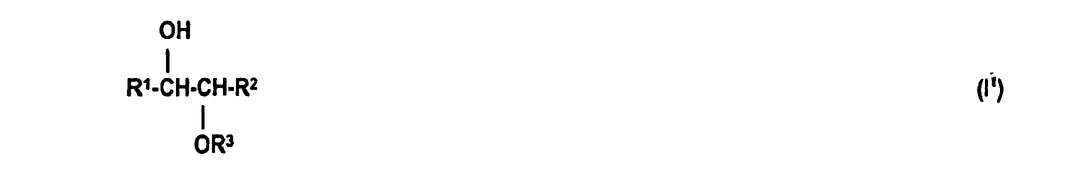 in der R¹ und R² für einen Alkylrest mit 4 bis 16 Kohlenstoffatomen steht, mit der Maßgabe, daß die Summe der Kohlenstoffatome von R¹ und R² im Bereich von 4 bis 16 liegt und R3 für einen Alkyl- und/oder Alkenylrest mit 1 bis 18 Kohlenstoffatomen steht und
(b) 0,1 bis 30 Gew.-% lipophile Feststoffe enthalten, mit der Maßgabe, daß sich die Mengenangaben mit Wasser und weiteren Hilfs- und Zusatzstoffen zu 100 Gew.-% ergänzen.

## Claims

1. The use of hydroxyethers corresponding to formula (I): 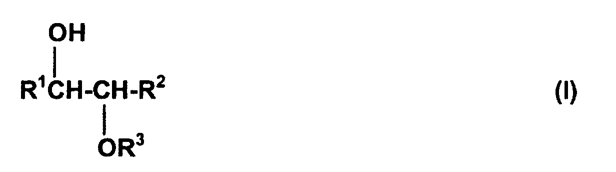 in which R¹ and R² represent an alkenyl group containing 4 to 16 carbon atoms, with the proviso that the sum of the carbon atoms of R¹ and R² is in the range from 4 to 16, and R³ is an alkyl and/or alkenyl group containing 1 to 18 carbon atoms and/or the residue of a polyol containing 2 to 18 carbon atoms and 2 to 10 hydroxyl groups,
as oil components for the production of cosmetic and/or pharmaceutical preparations for improving the feeling of smoothness of the skin.

2. The use claimed in claim 1, **characterized in that** lipophilic solids selected from the group consisting of UV filters, waxes, fats, stabilizers, pearlizing waxes, polymers and/or partial glycerides are dispersed in the oil components.

3. Preparations according to at least one of claims 1 to 2, **characterized in that** - based on the final concentration - they contain
(a) 1 to 60% by weight of hydroxyethers corresponding to formula (I'): 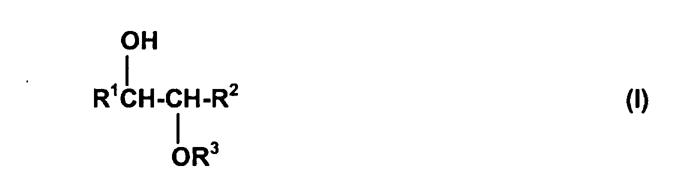 in which R¹ and R² represent an alkenyl group containing 4 to 16 carbon atoms, with the proviso that the sum of the carbon atoms of R¹ and R² is in the range from 4 to 16, and R³ is an alkyl and/or alkenyl group containing 1 to 18 carbon atoms, and
(b) 0.1 to 30% by weight of lipophilic solids, with the proviso that the quantities shown add up to 100% by weight with water and other auxiliaries and additives.

## Revendications

1. Utilisation d'hydroxyéthers de formule (I) 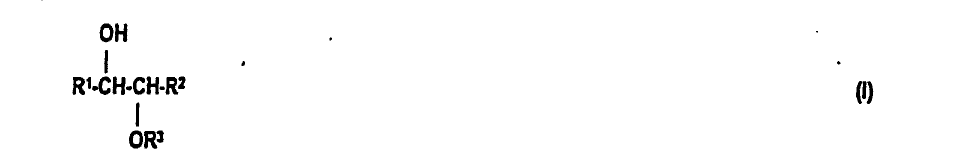 dans laquelle R¹ et R² représentent un reste alkyle ayant de 4 à 16 atomes de carbone avec la précision que la somme des atomes de carbone de R¹ et R² se situe dans la plage de 4 à 16,
et R³ représente un reste alkyle et/ou alkényle ayant de 1 à 18 atomes de carbone et/ou le reste d'un polyol ayant de 2 à 18 atomes de carbone et de 2 à 10 groupes hydroxyle
en tant que composés huileux pour la production de préparations cosmétiques et/ou pharmaceutiques en vue de l'amélioration du caractère lisse de la peau au toucher.

2. Utilisation selon la revendication 1,
**caractérisée en ce que**
dans les composés huileux on disperse des substances solides lipophiles choisies dans le groupe formé des filtres protecteurs contre la lumière UV, des cires, des graisses, des agents stabilisants, des cires à éclat nacré, des polymères et/ou des glycérides partiels.

3. Produits selon au moins l'une des revendications 1 à 2,
**caractérisés en ce qu'**
ils contiennent rapporté à la concentration finale
a) de 1 à 60 % en poids d'hydroxyéthers de formule (I') 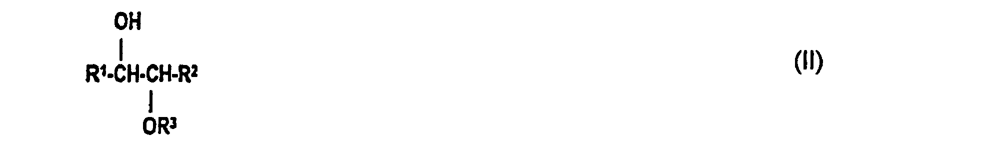 dans laquelle R¹ et R² représentent un reste alkyle ayant de 4 à 16 atomes de carbone avec la précision que la somme des atomes de carbone de R¹ et de R² se situe dans la plage de 4 à 16, et R³ représente un reste alkyle et/ou alkényle ayant de 1 à 18 atomes de carbone et
b) de 0,1 à 30 % en poids de substances solides lipophiles avec la précision que les données en quantités se complètent avec de l'eau et d'autres adjuvants et additifs à 100 % en poids.
